# EUROPEAN PATENT APPLICATION

(11) **EP 2 979 661 A1**
(43) Date of publication of application: **03.02.2016**
(21) Application number: 14773112.9
(22) Date of filing: 12.03.2014
(51) Int. Cl.: A61C 13/34, A61C 13/20, A61K 6/00

(54) **GYPSUM POWDER FOR DENTAL USE**

(30) Priority: 28.03.2013 JP 2013068560
(71) Applicant: GC Corporation, Tokyo 113-0033 (JP)
(72) Inventor: MORI, Daizaburo, Tokyo 174-8585 (JP); FUKUSHIMA, Emiko, Tokyo 174-8585 (JP); HORIUCHI, Haruhiko, Tokyo 174-8585 (JP)
(74) Representative: Müller-Boré & Partner Patentanwälte PartG mbB
(86) International application number: PCT/JP2014/056469
(87) International publication number: WO 2014/156643

(57) **Abstract**

The problem to be solved is to provide a dental gypsum powder capable of suppressing mixing of bubbles in a kneaded object while including a resin component. A dental gypsum powder includes 0.5 to 4 wt.% of a solid component included in acrylic polymer dispersion; and 0.001 to 0.2 wt.% of cyclic siloxane including 3 to 10 SiO(CH₃)₂ units. More preferably, it includes 1 to 3 wt.% of the solid component included in the acrylic polymer dispersion.

## Description

### BACKGROUND OF THE INVENTION

### 1. Field of the Invention

The present invention relates to a dental gypsum powder for which chipping (edge chipping) resistance is improved when cutting a cured body.

### 2. Description of the Related Art

In a dental field, dental gypsum is used as a mold material when manufacturing various dental prostheses that are attached in a mouth such as an inlay, a crown, a bridge, a partial denture, a complete denture or the like.

Generally, the dental gypsum includes α hemihydrate gypsum (hard gypsum) and/or β hemihydrate gypsum (calcined gypsum), each of which is hemihydrate gypsum, as a main constituent, and is provided in a form of a powder. A gypsum model (a working model or a jaw model) is manufactured by kneading the dental gypsum powder with water, filling it in a dental flask to invest a wax denture and curing it. Then, various dental prostheses are manufactured based on this gypsum model. As such, as the dental gypsum is a base in manufacturing a dental prosthesis, and a high accuracy of dimension is required for the dental prosthesis, it is strongly required for the dental gypsum that the dimensional change before and after curing is small and the surface quality of the cured body is glossy.

As problems regarding the dental gypsum, there have conventionally been problems that the gypsum model is easily damaged or chipping (edge chipping) easily occurs when releasing the gypsum model from a dental impression material or cutting the model. Thus, in order to prevent damage or chipping, a gypsum composition is disclosed that includes calcined gypsum, a fibrous antibacterial agent and resin as essential constituents (see Patent Document 1, for example). However, as this gypsum composition uses the fibrous antibacterial agent, there have been problems that operational feeling in kneading is bad and bubbles are easily mixed into a kneaded object. If bubbles are mixed in the kneaded object, the accuracy of the manufactured dental prosthesis is largely lowered.

Further, other than the dental field, in a field of a curable resin composition that is applied as a joint portion between stones or ceramics materials, techniques are disclosed that are capable of obtaining a design similar to mortars and appropriate physical properties by blending acrylic polymer dispersion as a resin component (see Patent Documents 2 and 3, for example). However, there have been problems that such a resin component cannot be applied to the dental gypsum because if such a resin component is used for the dental gypsum, the mixing water ratio becomes extremely low, the viscosity of the paste becomes high, and bubbles are mixed in the kneaded object.

Japanese Laid-open Patent Publication No. 2000-233962

Japanese Laid-open Patent Publication No. 2006-037034

Japanese Laid-open Patent Publication No. 2006-037039

The problem to be solved by the present invention is to provide a dental gypsum powder capable of suppressing mixing of bubbles in a kneaded object while including a resin component.

After having diligently studied in order to solve the above problem, the present inventors have found that the above problem can be solved by blending a combination of a specific resin component and siloxane in a dental gypsum powder to complete the present invention.

In other words, the dental gypsum powder of the invention is a dental gypsum powder characterized in including 0.5 to 4 wt.% of a solid component included in acrylic polymer dispersion, and 0.001 to 0.2 wt.% of cyclic siloxane including 3 to 10 SiO(CH₃)₂ units.

According to the dental gypsum powder of the invention, as it is possible to suppress mixing of bubbles in a kneaded object while including a resin component, a gypsum cured body can be manufactured which has a good chipping (edge chipping) resistance when being cut and in which bubbles are not mixed.

### DETAILED DESCRIPTION OF THE PREFERRED EMBODIMENTS

Hereinafter, embodiments of the invention are explained in detail. The dental gypsum powder of the invention includes acrylic polymer dispersion as a resin component. The acrylic polymer dispersion is dispersion in which acrylic polymer particles are dispersed in aqueous solvent. As the acrylic polymer, a copolymerized compound of a (meth)acrylic acid ester monomer including one (meth)acryloyl group and a (meth)acrylic acid ester monomer including two or more (meth)acryloyl groups may be used.

Specifically, acrylic acid alkyl ester copolymer, acrylonitrile·acrylic acid alkyl ester copolymer, acrylic acid·methacrylic acid·acrylic acid alkyl ester copolymer, acrylonitrile·acrylic acid·acrylic acid alkyl ester copolymer, styrene·acrylic acid alkyl ester copolymer, styrene·methacrylic acid alkyl ester·acrylic acid alkyl ester copolymer, styrene·acrylonitrile·acrylic acid alkyl ester copolymer, styrene·acrylonitrile·methacrylic acid alkyl ester·acrylic acid alkyl ester copolymer or the like may be raised, and these may be used solely or two or more of these may be used together. Among these, acrylic acid alkyl ester copolymer, acrylonitrile·acrylic acid alkyl ester copolymer, acrylic acid·methacrylic acid·acrylic acid alkyl ester copolymer and acrylonitrile·acrylic acid·acrylic acid alkyl ester copolymer are preferable due to a reason that chipping resistance of the dental gypsum after being cured is good.

The ratio of a solid component (an acrylic polymer component) in the acrylic polymer dispersion that is blended in the dental gypsum powder of the invention is 0.5 to 4 wt.% in the dispersion. When the ratio is less than 0.5 wt.%, strength of the gypsum after being cured cannot be increased and chipping resistance is not improved. Further, when the ratio exceeds 4 wt.%, bubbles are mixed. Preferably, the ratio is 0.5 to 3wt.%, and more preferably, 1 to 3 wt.%.

The dental gypsum powder of the invention includes 0.001 to 0.2 wt.% of cyclic siloxane including 3 to 10 SiO(CH₃)₂ units. By blending this specific cyclic siloxane, it is possible to suppress mixing of bubbles when kneading the dental gypsum powder and water. Further, as the effect can be obtained by a small blending amount, this does not exert a bad influence on various performances of the gypsum cured body. If the content of the cyclic siloxane including 3 to 10 SiO(CH₃)₂ units is less than 0.001 wt.%, a desired effect cannot be obtained, and if the content exceeds 0.2 wt.%, easiness to be kneaded as dental gypsum becomes worse.

The cyclic siloxane including 3 to 10 SiO(CH₃)₂ units includes 3 to 10 SiO(CH₃)₂ units (hereinafter, referred to as "D units" as well), and is hexamethylcyclotrisiloxane when including 3 D units, is octamethylcyclotetrasiloxane when including 4 D units, and is decamethylcyclopentasiloxane when including 5 D units, for example. The preferable number of D units is 3 to 5, and in particular, octamethylcyclotetrasiloxane including 4 D units is most preferable due to its easiness to be kneaded and its high effect. Further, it is possible to use mixture of two or more kinds of cyclic siloxane whose D units are different.

For the dental gypsum powder of the invention, it is possible to use conventional silicone oil together. As the silicone oil, dimethyl silicone oil or methylphenyl silicone oil may be raised.

The dental gypsum powder of the invention is calcium sulfate (CaSO₄), and is obtained by blending the above described cyclic siloxane to a conventional dental gypsum powder including hemihydrate gypsum as a main constituent. As the hemihydrate gypsum, α hemihydrate gypsum, β hemihydrate gypsum, or a mixture of powders of α hemihydrate gypsum and β hemihydrate gypsum may be used.

For the dental gypsum powder, although operativity and physical properties are set in accordance with various intended use such as a model material, a denture investment material or the like, as curing speed of the hemihydrate gypsum itself is fast, generally, curing retarder is added. As the curing retarder, a known component such as citrate, borate, carboxylate, acetate or the like, water-soluble polymer such as starch, gum arabic, carboxymethyl cellulose, gelatin or the like may be used, and generally, 0.00001 to 0.2 wt. part is blended with respect to 100 wt. part of hemihydrate gypsum powder.

When it is necessary to sensitively adjust curing time, curing accelerator may be further used. As the curing accelerator, a known component such as sodium chloride, potassium sulfate or the like, gypsum dihydrate fine powder or the like may be used, and generally, when blending, 0.001 to 2 wt. part is blended with respect to 100 wt. part of hemihydrate gypsum powder.

Curing expansion inhibitor such as potassium tartrate or the like may be blended in the dental gypsum powder of the invention. It is preferable that 0.01 to 1 wt. part of the curing expansion inhibitor is blended with respect to 100 wt. part of hemihydrate gypsum powder. In addition, a known coloring agent or a lightening material may be blended in accordance with necessity.

A conventionally used water reducing admixture may be blended in the dental gypsum powder of the invention.

As the dental gypsum powder of the invention is obtained by adding and blending the acrylic polymer dispersion and the cyclic siloxane to the powder component of the dental gypsum, it is in form of powder, similar to the conventional dental gypsum, and a gypsum model may be manufactured by adding an appropriate amount of water to the powder, for example, 18 to 28 wt. part of water with respect to 100 wt. part of the powder to be paste, shaping in a desired shape and curing it. In other words, it is unnecessary to further use special emulsifier or special kneading solution.

Hereinafter, examples of the dental gypsum powder of the invention are described and explained in detail. Examples (Raw powder A in which cyclic siloxane blended silicone oil is mixed)

5 g of silicone oil (polydimethylsiloxane) including 2 wt.% of hexamethylcyclotrisiloxane and 10 wt.% of octamethylcyclotetrasiloxane is mixed into 95 g of α hemihydrate gypsum powder and stirred for 1 hour to prepare a powder including 5 wt.% of silicone oil (0.6 wt.% of cyclic siloxane). This is referred to as a raw powder A.

### (Raw powder B in which cyclic siloxane blended silicone oil is mixed)

10 g of silicone oil (polydimethylsiloxane) including 15 wt.% of octamethylcyclotetrasiloxane was mixed into 90 g of α hemihydrate gypsum powder by the above described method to prepare a powder including 10 wt.% of silicone oil (1.5 wt.% of cyclic siloxane). This is referred to as a raw powder B.

Components were mixed in accordance with ratios illustrated in Table 1 using a mixer, and a dental gypsum powder of each examples and comparative examples was manufactured.

### (Evaluation of fluidity)

100 g of the dental gypsum powder manufactured in each of examples and comparative examples and 20 g of water were put in a rubber bowl, mixed by a gypsum spatula, the kneaded mud is filled into a ring designated by JIS T6601 "Dental gypsum-bonded casting investments", the ring was detached two minutes after starting kneading, vibrated for 5 seconds and an expanded value (fluidity) of the kneaded mud was measured. Marks indicating evaluations in Table 1 are as follows.

| | |
|---|---|
| ○ | exceeds 60 mm |
| Δ | 40 to 60 mm |
| × | less than 40 mm |

Results are illustrated in Table 1.

### (Evaluation of chipping resistance)

100 g of the dental gypsum powder manufactured in each of examples and comparative examples and 20 g of water were put in a rubber bowl, mixed by a gypsum spatula, the mixed paste was flew into a mold having a diameter of 25 mm and a height of 50 mm and stood for 30 minutes. For a surface of the cured dental gypsum, a degree of scratches was evaluated using a scratching tester. Marks indicating evaluations in Table 1 are as follows.
○ : chippings or the like were not seen at peripheral of a scratch mark, and the scratch mark was a fine line.
Δ : chippings were seen at peripheral of a scratch mark and the scratch mark became a bit bold line.
× : a lot of chippings were seen at peripheral of a scratch mark, and the scratch mark was not a straight line.

Results are illustrated in Table 1.

### (Evaluation of defoaming property)

100 g of the dental gypsum powder manufactured in each of examples and comparative examples and 20 g of water were put in a rubber bowl, mixed by a gypsum spatula, the mixed paste was flew into a mold having a diameter of 25 mm and a height of 50 mm and stood for 30 minutes. After curing the dental gypsum, the model was cut into half, and degree of bubbles confirmed at the cut surface was evaluated by a visual observation. For the case when the bubbles were not seen similarly as for a conventional gypsum cured body, it was evaluated as "○". When the bubbles were clearly mixed, it was evaluated as "×". Results are illustrated in Table 1.

**[Table 1]**

| | | Example 1 | | Example 2 | | Example 3 | | Example 4 | | Comparative example 1 | | Comparative example 2 | | Comparative example 3 | Comparative example 4 | Comparative example 5 | Comparative example 6 |
|---|---|---|---|---|---|---|---|---|---|---|---|---|---|---|---|---|---|
| *α* hemihydrate gypsum | | 98.4 | | 96.7 | | 97 | | 98.5 | | 98.8 | | 96 | | 100 | 98 | 97 | 95 |
| Raw powder A | polydimethylsiloxane | 0.1 | 0.0044 | 0.3 | 0.0132 | | | | | 0.1 | 0.0044 | | | | | | |
| | *octamethylcyclotetrasiloxane | | 0.0005 | | 0.0015 | | | | | | 0.0005 | | | | | | |
| | *hexamethylcyclotrisiloxane | | 0.0001 | | 0.0003 | | | | | | 0.0001 | | | | | | |
| | α hemihydrate gypsum | | 0.095 | | 0.285 | | | | | | 0.095 | | | | | | |
| Raw powder B | polydimethylsiloxane | | | | | 2 | 0.17 | 0.5 | 0.0425 | | | 4 | 0.34 | | | | |
| | *octamethylcyclotetrasiloxane | | | | | | 0.03 | | 0.0075 | | | | 0.06 | | | | |
| | α hemihydrate gypsum | | | | | | 1.8 | | 0.45 | | | | 3.6 | | | | |
| acrylic polymer dispersion | Mowinyl-Powder LDM7300P | 1.5 | | | | | | | | | | | | | | | 5 |
| | Mowinyl-Powder LDM7000P | | | 3 | | | | | | | | | | | | 3 | |
| | Acronal YJ1555D | | | | | 1 | | | | | | | | | 2 | | |
| | Acronal 430P | | | | | | | 1 | | | | | | | | | |
| Water reducing admixture | MelmentF10 | 0.3 | | 0.3 | | 0.3 | | 0.3 | | 0.2 | | 0.2 | | 0.2 | 0.3 | 0.3 | 0.3 |
| FLUIDITY | | ○ | | ○ | | ○ | | ○ | | ○ | | ○ | | ○ | ○ | Δ | Δ |
| CHIPPING RESISTANCE | | ○ | | ○ | | ○ | | ○ | | × | | × | | × | ○ | ○ | ○ |
| DEFOAMING PROPERTY | | ○ | | ○ | | ○ | | ○ | | ○ | | ○ | | ○ | × | × | × |

| | | | | | | | | | | | | | | | | | |
|---|---|---|---|---|---|---|---|---|---|---|---|---|---|---|---|---|---|
| * is cyclic siloxane including 3 to 10 SiO(CH₃)₂ units UNIT: wt.% | | | | | | | | | | | | | | | | | |

In the table, Mowinyl-Powder LDM7300P and Mowinyl-Powder LDM7000P are acrylic polymer dispersion manufactured by Nippon Synthetic Chemical Industry Co., Ltd., Acronal YJ1555D and Acronal 430P are acrylic polymer dispersion manufactured by BASF Ltd. and MelmentF10 is water reducing admixture manufactured by BASF Ltd.

As is clearly understood from Table 1, for examples 1 to 4, the evaluations are "○" for all of the fluidity, the chipping resistance and the defoaming property. On the other hand, for all of comparative examples 1 to 3, in each of which acrylic polymer dispersion was not blended, the evaluations are "×" for the chipping resistance, and for all of comparative examples 4 to 6, in each of which cyclic siloxane including 3 to 10 SiO(CH₃)₂ units was not blended, the evaluations are "×" for the defoaming property and it can be understood those are not preferable for the dental gypsum powder.

## Claims

1. A dental gypsum powder comprising:
0.5 to 4 wt.% of a solid component included in acrylic polymer dispersion; and
0.001 to 0.2 wt.% of cyclic siloxane including 3 to 10 SiO(CH₃)₂ units.

2. The dental gypsum powder according to claim 1,
wherein the solid component included in the acrylic polymer dispersion is a polymer of one or more selected from a group consisting of acrylic acid alkyl ester copolymer, acrylonitrile·acrylic acid alkyl ester copolymer, acrylic acid·methacrylic acid·acrylic acid alkyl ester copolymer, acrylonitrile·acrylic acid·acrylic acid alkyl ester copolymer, styrene·acrylic acid alkyl ester copolymer, styrene·methacrylic acid alkyl ester·acrylic acid alkyl ester copolymer, styrene·acrylonitrile·acrylic acid alkyl ester copolymer and styrene·acrylonitrile·methacrylic acid alkyl ester·acrylic acid alkyl ester copolymer.
